# EUROPÄISCHE PATENTSCHRIFT

(11) **EP 3 684 286 B1**
(45) Veröffentlichungstag und Bekanntmachung des Hinweises auf die Patenterteilung: **06.01.2021**
(21) Anmeldenummer: 19765679.6
(22) Anmeldetag: 04.09.2019
(51) Int. Cl.: A61B 50/22, A61B 50/30, A61B 50/00

(54) **VERPACKUNG MIT EINER HALTEVORRICHTUNG ZUM HALTEN EINES MEDIZINTECHNISCHEN WERKZEUGS**
PACKAGING HAVING A HOLDING DEVICE FOR HOLDING A MEDICAL TOOL
EMBALLAGE DOTÉ D'UN DISPOSITIF DE RETENUE DESTINÉ À MAINTENIR UN OUTIL UTILISÉ EN TECHNIQUE MÉDICALE

(30) Priorität: 05.09.2018 DE 102018121683
(43) Veröffentlichungstag der Anmeldung: 29.07.2020
(73) Patentinhaber: Aesculap AG, 78532 Tuttlingen (DE)
(72) Erfinder: LENZENHUBER, Frederick, 78532 Tuttlingen (DE); HÖGERLE, Roland-Alois, 78532 Tuttlingen (DE)
(74) Vertreter: Winter, Brandl, Fürniss, Hübner, Röss, Kaiser, Polte - Partnerschaft mbB
(86) Internationale Anmeldenummer: PCT/EP2019/073577
(87) Internationale Veröffentlichungsnummer: WO 2020/049042

(56) Entgegenhaltungen:
- EP-A1- 3 338 725
- DE-A1-102005 048 211
- DE-U1-202011 050 118
- FR-A1- 2 999 159

## Beschreibung

Die vorliegende Erfindung betrifft eine Verpackung, insbesondere Sterilverpackung, mit einer Haltevorrichtung zum lagebestimmten Halten eines medizintechnischen Werkzeugs der Schaftbauart mit einem Werkzeugschaft mit einem vor anwenderseitigen Kontakt zu schützenden Arbeitsende/Effektor in einem Aufnahmeraum der Verpackung und einer den Aufnahmeraum für die Haltevorrichtung und das darin gehaltene Werkzeug ausbildenden Umverpackung, wobei die Haltevorrichtung Haltemittel/eine Haltestruktur zur positionsbestimmten Aufnahme/Lagerung des Werkzeugs aufweist.

Es sind verschiedene Verpackungen und Vorrichtungen für medizintechnische Werkzeuge bekannt. Verpackungen von medizintechnischen Werkzeugen mit spitzen und/oder scharfen Arbeitsenden, beispielsweise in Form von Schneid- oder Sägekanten, Fräsköpfen und Bohrern oder Gewindeabschnitten, unterliegen besonderen Anforderungen. Denn neben dem Schutz von Anwendern vor Verletzungen vor und insbesondere nach einer Verwendung des Werkzeugs ist außerdem ein Schutz des verpackten Werkzeugs vor ungewünschten Einflüssen wie Verschmutzung und Beschädigung sicherzustellen. Insbesondere ist sicherzustellen, dass mit einem Patienten in Kontakt gelangende Arbeitsenden des Werkzeugs, die in der Regel scharfkantig sind, nicht anwenderseitig berührt werden. Zwar wird in Gebrauchsanweisungen immer wieder darauf hingewiesen, dass ein Berühren der Arbeitsenden unbedingt zu vermeiden ist, aber bei üblichen Operationsabläufen kann es nur allzu leicht vorkommen, dass der Anwender, zum Beispiel ein Chirurg oder medizinisches Hilfspersonal, dennoch versehentlich die Arbeitsenden von Werkzeugen, Klingen und dergleichen beim Einbringen in ein Griffstück, ein Trägerteil oder eine Antriebseinheit für das Werkzeug berührt. Dadurch kann es zum einen zu einer unter Umständen unbemerkt bleibenden Beschädigung von Schutzkleidung, wie Gummihandschuhen, kommen, was ein erhebliches Risiko für den Anwender beinhaltet. Zum anderen kann das Arbeitsende des Werkzeugs mit Fremdpartikeln kontaminiert werden, was wiederum ein erhebliches Risiko für den Patienten beinhaltet.

Aus dem Stand der Technik sind Verpackungen bekannt, in denen das medizintechnische Werkzeug fest und/oder lagedefiniert gehalten ist. Ein Beispiel für solche Verpackungen ist eine Blisterverpackung mit einer Klemmung im Kupplungsbereich des Werkzeugs, um dieses in einer definierten Lage in der Verpackung zu positionieren und zu halten. Eine solche Verpackung ist zum Beispiel aus der DE 10 2013 004 168 A1 offenbart, bei der das Operationswerkzeug unter sterilen Bedingungen in einer Kunststoffverpackung verpackt ist, die Verpackung mindestens zweiteilig ausgebildet ist und aus einer inneren Schutzverpackung für empfindliche Teile des Operationswerkzeugs und einer die Schutzverpackung aufnehmenden, siegelfähigen Blisterverpackung besteht. Außerdem wird ein Verfahren zur Verpackung von sterilen Operationswerkzeugen vorgeschlagen, bei dem das Operationswerkzeug unter sterilen Bedingungen in der Kunststoffverpackung verpackt wird, wobei in einem ersten Schritt zunächst das Operationswerkzeug mindestens teilweise und insbesondere mit seinen empfindlichen Schneidkanten und Arbeitsflächen in der ersten Schutzverpackung verpackt wird und die Schutzverpackung zusammen mit dem dort verpackten Operationswerkzeug in eine zweite Blisterverpackung verbracht wird, dort lagengesichert festgelegt wird und danach mit einer Siegelfolie verschlossen wird. Bei dieser Verpackung besteht der Nachteil, dass zwar die Wirkkanten des Werkzeugs sehr gut geschützt sind, das Werkzeug jedoch beim Einbringen in ein Griffstück die innere Schutzverpackung kontaktieren kann und so verunreinigt werden kann.

FR 2 999 159 A1 offenbart eine Verpackung gemäß dem Oberbegriff des Anspruchs 1. Insbesondere offenbart FR 2 999 159 A1 eine Blisterverpackung mit einer Haltevorrichtung zum Halten eines schaftartigen medizinischen Stifts/ Pins und einer Umverpackung. Die Haltevorrichtung weist dabei Haltemittel auf. Ein Ende des Stifts/ Pins liegt an einem hochstehenden Rand der Haltevorrichtung an.

Weiterer relevanter Stand der Technik findet sich in der DE 10 2005 048211 A1, der EP 3 338 725 A1 und der DE 20 2011 050118 U1. Dabei offenbart die DE 10 2005 048211 A1 eine chirurgische Lagerung, welche geeignet ist, ein chirurgisches Werkzeug zu halten. Die Lagerung weist dabei Haltemittel und Anschläge auf. Die EP 3 338 725 A1 offenbart eine Haltevorrichtung für ein schaftartiges medizinisches Werkzeug mit Haltemitteln und Anschlägen. Die DE 20 2011 050118 U1 offenbart ein chirurgisches Behälterinhaltdetektionssystem.

Bekannte Haltevorrichtungen von Verpackungen verhindern nicht sicher ein Berühren der Arbeitsenden. Ein weiterer Nachteil ist, dass zum Einbringen des Werkzeugs in Handstücke oder Antriebseinheiten weitere Hilfsmittel oder Werkzeuge notwendig sind, wenn ein direkter Kontakt des Anwenders mit dem Werkzeug verhindert werden soll, was die Handhabung deutlich erschwert.

Vor diesem Hintergrund liegt der vorliegenden Erfindung die Aufgabe zugrunde, die genannten Nachteile des Stands der Technik zu verringern, insbesondere eine Verpackung für ein medizintechnisches Werkzeug der Schaftbauart zur Verfügung zu stellen, mit der eine Handhabung des Werkzeugs insbesondere beim Einbringen in ein Griffstück oder eine Antriebseinheit einfach und sicher möglich ist und das Risiko von Kontamination des Werkzeugs und insbesondere dessen Arbeitsendes und gleichzeitig von Verletzungen von Anwendern minimiert werden kann.

Diese Aufgabe wird nach der vorliegenden Erfindung gelöst durch eine Verpackung nach Anspruch 1, also eine Verpackung, insbesondere Sterilverpackung, mit einer Haltevorrichtung zum lagebestimmten Halten eines medizintechnischen Werkzeugs mit einem Werkzeugschaft mit einem vor anwenderseitigen Kontakt zu schützenden Arbeitsende in einem Aufnahmeraum der Verpackung und einer den Aufnahmeraum für die Haltevorrichtung und das darin gehaltene Werkzeug ausbildenden Umverpackung, wobei die Haltevorrichtung Haltemittel zur positionsbestimmten Aufnahme des Werkzeugs und einen Anschlag für das Arbeitsende zum Positionieren des Werkzeugs in Richtung des Werkzeugschafts aufweist, und wobei der Anschlag zumindest teilweise oder abschnittsweise aus einem resorbierbarem Material besteht.

In anderen Worten ausgedrückt stellt die vorliegende Erfindung eine Werkzeugaufnahme oder ein Werkzeugauflager bereit, das dazu angepasst und vorgesehen ist, in einer Sterilverpackung aufgenommen zu werden. Das Werkzeugauflager hat zumindest eine, vorzugsweise mehrere (axial-) beabstandete Schaftaufnahmeklemmen (jeweils bestehend aus zwei federelastischen/federelastisch gelagerten Klemmarmen/Klemmbacken), die an einer Sockel- oder Grundplatte vorzugsweise stoffeinstückig angeordnet ist/sind und bevorzugt jeweils zumindest eine oder mehrere Ausbauchungsabschnitte zur (teileweise) umgreifenden Aufnahme eines Werkzeugschafts ausgebildet/ausbilden.

Zur Vermeidung einer Axialbewegung des Werkzeugs/Werkzeugschafts in der/den Werkzeugaufnahmeklemme(n) sind zwei (axial-) beabstandete Endanschläge vorgesehen, von denen bevorzugt wenigstens ein Endanschlag als separates Bauteil fertigt und an unterschiedlichen Positionen an der Grundplatte zur Anpassung an unterschiedliche Schaftlängen montierbar ist. Besonders vorteilhaft ist es, dass wenigstens ein Endanschlag, vorzugsweise der separate Endanschlag aus einem resorbierbaren Material besteht oder Teile davon aus resorbierbarem Material bestehen.

Unter einem Werkzeug im Sinne der Erfindung ist insbesondere ein chirurgisches Werkzeug / Operationswerkzeug mit einem in der Regel distalen, scharfkantigen Arbeitsende zum Erfüllen einer operativen Funktion und einem proximalen Koppelabschnitt / Koppelstruktur zum Koppeln des Werkzeugs mit einer Griffeinheit oder Griffstück, insbesondere einer Antriebsgriffeinheit, zu verstehen. Beispiele für solche Werkzeuge sind vor allem rotierende Werkzeuge sowie Sägewerkzeuge, unter anderem Bohrer, Fräser, Sägen, Schraubaufsätze, Schneidklingen oder Schleifaufsätze, die in allgemein bekannter Weise mit einer Griff- und/oder Antriebseinheit gekoppelt werden. Außerdem ist unter einem Werkzeug im Sinne der Erfindung eine Sprühdüse, eine HF-Spritze, eine Ultraschallklinge, etc. zu verstehen. Schließlich beinhaltet der Begriff "Werkzeug" im Sinne der Erfindung auch Implantate beliebiger Art und Form, wie zum Beispiel Knochen- und Gelenkimplantate oderteilimplantate, Stants, etc., die bei einer Entnahme aus der Haltevorrichtung mit einem Handhabungsmittel gekoppelt werden. Wirkabschnitte solcher Implantate, wie zum Beispiel Gewindeabschnitte oder ähnliches, stellen das Arbeitsende im Sinne der Erfindung dar.

Es ist ein besonderer Vorteil der Erfindung, dass die Haltevorrichtung einen die Lage des Werkzeugs in Richtung seines Schafts, also in Richtung von der proximalen Koppelstruktur zum distalen Arbeitsende, definierenden Anschlag aufweist. Der Anschlag besitzt eine Anlagefläche, an der das Arbeitsende des Werkzeugs bei der Entnahme aus der Haltevorrichtung in Anlage steht oder zur Anlage kommt. Somit ist das Werkzeug auch bei einer anwenderseitigen Entnahme aus der Haltevorrichtung in dieser Richtung lagepositioniert bzw. durch eine Anlage an dem Anschlag fixiert und kann ohne zusätzlich durch den Anwender gehalten werden zu müssen, in ein Griffstück oder eine Antriebseinheit eingebracht und mit dieser gekoppelt werden. Außerdem ist das vor anwenderseitiger Berührung zu schützende Arbeitsende des Werkzeugs von dem Anschlag abgedeckt, so dass das Risiko von Verletzungen minimiert ist. Eine besonders gute Abdeckung des Arbeitsendes kann erzielt werden, wenn der Anschlag zu allen Seiten über das Arbeitsende hinausragt. Man kann daher auch sagen, dass die Erfindung sicherstellt, dass das Arbeitsende des Werkzeugs beim Koppelprozess mit einem Griffstück nur vom Anschlag berührt werden kann.

Vorteilhafte Ausführungsformen der Erfindung sind in den Unteransprüchen beansprucht und werden nachfolgend näher erläutert.

Nach einer Ausführungsform kann der Anschlag zumindest teilweise oder abschnittsweise aus einem resorbierbaren Material bestehen, vorzugweise besteht er vollständig aus einem resorbierbaren Material. Dies bewirkt den Vorteil, dass das Arbeitsende des Werkzeugs bei einem Koppelprozess mit einem Griffstück oder einer Antriebseinheit an dem Anschlag anliegen kann, ohne dass bio-unverträgliches Material abgerieben wird, an dem Werkzeug anhaftet und versehentlich in den Körper eines Patienten eingebracht wird. Man kann auch sagen, dass derart sichergestellt wird, dass das Werkzeug beim Kupplungsprozess lediglich resorbierbares Material berührt und ein Anhaften von resorbierbarem Material ist unkritisch. Bei dem resorbierbaren Material im Sinne der Erfindung kann es sich um ein resorbierbares Biopolymer oder ein Copolymer davon, insbesondere um einen resorbierbaren Polyester, Gelatine, thermoplastische Stärke (TPS) oder Zusammensetzungen oder Mischungen daraus handeln. Vorzugsweise wird als resorbierbares Material im Sinne der Erfindung ein Polyaktid (PLA), insbesondere ein Polyaktid der chemischen Formel (C3H4O2)n-O-CO-CH(CH4), insbesondere ein Poly-L-Laktid (PLLA), ein Poly-D-Laktid (PDLA) oder ein Poly-DL-Laktid (PDLLA), PCL (Polycaprolactan), PHB (Polyhydroxybuttersäure) oder eine Zusammensetzung oder eine Mischung aus den vorstehend genannten Materialen verwendet. Diese resorbierbaren Materialien sichern nicht nur einen kontrollierbaren und sicheren biologischen Abbau im Körper, sondern sind außerdem in höchstem Maß gewebeverträglich. Im Falle von PLA spaltet im Körper des Patienten vorhandenes Wasser die Polymerketten dieses Materials auf und der menschliche Stoffwechsel wandelt die D- und L-Laktide letztlich in Kohlendioxid und Wasser um, so dass versehentlich in den Körper des Patienten verbrachte Materialpartikel in besonders vorteilhafter Weise rückstandsfrei abgebaut werden. Es liegt im Rahmen der Erfindung, dass die gesamte Haltevorrichtung teilweise oder vollständig aus resorbierbarem Material besteht.

Nach einer weiteren Ausführungsform ist der Anschlag separat von der Haltevorrichtung ausgebildet. Er kann insbesondere in einer Mehrzahl unterschiedlicher Positionen an der Haltevorrichtung, insbesondere in unterschiedlichen Positionen in Richtung des Werkzeugschafts, anordbar sein und angeordnet werden. Auf diese Weise kann durch die Erfindung eine Haltevorrichtung zur Verfügung gestellt werden, die einfach auf die jeweilige Länge des darin anzuordnenden Werkzeugs einzustellen und damit hochflexibel für unterschiedliche Werkzeuge mit unterschiedlicher Geometrie einsetzbar ist.

Eine weitere Ausführungsform der Erfindung ist dadurch gekennzeichnet, dass diese einen weiteren Anschlag für eine dem Arbeitsende gegenüberliegende Koppelstruktur des Werkzeugs aufweist. Dieser weitere Anschlag kann auch als proximaler Anschlag bezeichnet werden (der erste Anschlag kann als distaler Anschlag bezeichnet werden) und dient zum Positionieren des Werkzeugs in Richtung des Werkzeugschafts, insbesondere in proximaler Richtung. Er überdeckt die Koppelstruktur und ragt dabei ggf. zu allen Seiten über diese hinaus, so dass die Koppelstruktur besonders gut geschützt ist. Der weitere Anschlag kann in vorteilhafter Weise mittels einer Sollbruchstelle mit der Haltevorrichtung verbunden sein. Durch Brechen der Sollbruchstelle kann er einfach von der Haltevorrichtung getrennt und entfernt werden, so dass die Koppelstruktur des Werkzeugs nach Entfernen des weiteren Anschlags zum Koppeln mit einem Griffstück oder einer Antriebseinheit zugänglich ist.

Die Haltevorrichtung kann eine Grundplatte aufweisen. Diese kann insbesondere derart ausgebildet sein, dass sie eine Standfläche zum lagestabilen Aufstellen der Haltevorrichtung mit einem darin gehaltenen Werkzeugs, insbesondere in einem aus der Verpackung ausgepackten Zustand, und/oder eine Anlagestruktur zur Anlage an der Verpackung ausbildet. Neben der Standfläche kann die Haltevorrichtung weitere Anlagestrukturen oder-abschnitte aufweisen, die mit der Verpackung zusammenwirken und an dieser anliegen, so dass eine eindeutige und stabile Lagepositionierung in der Aufnahme der Verpackung gewährleistet ist.

Die Haltevorrichtung kann nach einer Ausführungsform der Erfindung zumindest zwei voneinander beabstandete Haltestrukturen zum lagebestimmten Positionieren des Werkzeugs aufweisen. Bevorzugt sind diese jeweils an der Grundplatte angeordnet und können sich von dieser in den Aufnahmeraum hinein erstrecken. Neben der Funktion des Haltens des Werkzeugs können die Haltestrukturen Anlagen ausbilden, mit denen die Haltevorrichtung an der Verpackung anliegt, vorzugsweise derart, dass sie in der Aufnahme der Verpackung lagegesichert oder lagefixiert ist. Eine der Haltestrukturen kann endseitig an der Haltevorrichtung, insbesondere an der Grundplatte, angeordnet sein. Eine andere der Haltestrukturen kann im Wesentlichen mittig an der Haltevorrichtung, insbesondere an der Grundplatte, angeordnet sein. Dies ermöglicht eine besonders stabile Halterung des Werkzeugs in der Haltevorrichtung.

Vorzugsweise ist eine der Haltestrukturen zum lagebestimmten Positionieren des Werkzeugs relativ zur Haltevorrichtung in einer ersten Richtung ausgebildet. Die andere der Haltestrukturen kann zum lagebestimmten Positionieren des Werkzeugs relativ zur Haltevorrichtung in einer zweiten Richtung und einer dritten Richtung ausgebildet sein, wobei die erste, zweite und dritte Richtung zueinander orthogonal sind. Durch eine derartige Führung ist das Werkzeug einerseits stabil in der Haltevorrichtung gehalten, andererseits kann es besonders einfach durch einen Anwender in der Haltevorrichtung angeordnet und aus der Haltevorrichtung entnommen werden. Es ist besonders vorteilhaft, wenn nach einer weiteren Ausführungsform der Verpackung zumindest eine der Haltestrukturen mit dem Werkzeug einen Formschluss und/oder einen Kraftschluss ausbildet, insbesondere mit einer Koppelstruktur des Werkzeugs.

Zumindest eine der Haltestrukturen, insbesondere beide Haltestrukturen, kann bzw. können jeweils zwei einander gegenüberliegende Haltearme aufweisen. Zwischen diesen ist jeweils ein Schlitz zur Aufnahme und Halterung des Werkzeugs ausgebildet. Die Haltearme können im Wesentlichen senkrecht zur Grundplatte der Haltevorrichtung angeordnet sein. Sie können in besonders vorteilhafter Weise als Klemmarme ausgebildet sein und insbesondere in einer Richtung quer zum Schlitz federnde Eigenschaften aufweisen. Ein Entnehmen sowie ein Anordnen des Werkzeugs in den Haltestrukturen ist besonders einfach möglich, wenn nach einer Form der Erfindung sich der Schlitz von proximal der Basis in Richtung distal der Basis aufweitet. Dies begünstigt eine weitgehend gefahrlose Handhabung sowohl von ungebrauchten als auch von gebrauchten Instrumenten, so dass insbesondere eine verletzungsfreie Entsorgung von Werkzeugen in der Halterung verbessert und die dabei grundsätzlich bestehende Infektionsgefahr verringert wird. Nach einer Fortbildung der Erfindung können die dem Schlitz zugewandten Seiten der Haltearme mit Einbuchtungen versehen sein. Solche Einbuchtung können in vorteilhafter Weise Rastbuchten für das in den Schlitz eingebrachte Werkzeug bilden, so dass dieses sicher und dennoch für einen Nutzer einfach entnehmbar in den Haltestrukturen gehalten ist.

Eine besonders nutzerfreundliche, da Verletzungen eines Nutzers an dem in der Haltevorrichtung vermeidende Ausführungsform der Erfindung sieht vor, dass die Haltevorrichtung eine Schutzlasche aufweist, die von der Grundplatte in den Aufnahmeraum hineinragt und ein distales Ende des Werkzeugs oder eine scharfkantige Struktur des Werkzeugs berührungslos abdeckt. Auf diese Weise ist ein Anwender beim Handhaben der Haltevorrichtung mit dem darin gehaltenen Werkzeug vor Verletzungen durch gegebenenfalls scharfe Kanten des Werkzeugs geschützt.

Eine weitere Ausführungsform ist dadurch gekennzeichnet, dass in der Grundplatte ein Mehrzahl von zueinander beabstandeten Ausnehmungen ausgebildet ist und der Anschlag für das Arbeitsende einen dazu passenden Zapfen aufweist, so dass der Anschlag durch Einbringen des Zapfens in eine der Ausnehmungen in unterschiedlichen Positionen an der Grundplatte anordbar ist. Dies ermöglicht in vorteilhafter Weise eine einfache Einstellbarkeit der Haltevorrichtung an unterschiedliche Werkzeuggeometrien.

Eine Ausführungsform der Erfindung ist dadurch gekennzeichnet, dass die Haltevorrichtung lagebestimmt in dem Aufnahmeraum angeordnet und/oder gehalten ist. Sie dient insbesondere dazu, das Werkzeug zu halten, insbesondere lagebestimmt zu halten und im Aufnahmeraum derart zu positionieren, dass ein Kontakt von scharfen Arbeitsenden mit dem Verpackungsmaterial vermieden wird und diese von der Verpackung beabstandet sind. Durch Vorsehen einer solchen Haltevorrichtung kann ein Kontakt des Werkzeugs mit Teilen der Verpackung verhindert oder zumindest sicher minimiert werden, so dass Abrieb von Verpackungsmaterial durch das Werkzeug verhindert werden kann. Die Umverpackung kann außerdem zumindest teilweise, vorzugsweise vollständig, aus einem resorbierbaren Material bestehen. Der Aufnahmeraum kann durch die Umverpackung geschlossenen ausgebildet ist, insbesondere hermetisch dicht. Er kann außerdem steril ausgebildet sein.

Nach einer Ausführungsform der Erfindung ist die Haltevorrichtung separat von der Umverpackung ausgebildet. Daher kann das in der Haltevorrichtung gehaltene Werkzeug zusammen mit dieser aus der Umverpackung entnommen werden und insbesondere auch nach dem Auspacken bis zum letztlichen Gebrauch in der Haltevorrichtung verbleiben. Vorzugsweise sind Verpackung und Haltevorrichtung derart ausgebildet, dass ein Entnehmen durch Handhabung der Haltevorrichtung und ohne direkte Kontaktierung des Werkzeugs erfolgen kann, so dass die Wahrscheinlichkeit einer Kontaminierung des Werkzeugs einfach und sicher minimiert, wenn nicht sogar vermieden werden kann. Außerdem kann das Werkzeug zusammen mit der Haltevorrichtung auf einem Instrumentiertisch platziert werden, ohne es lose ablegen zu müssen. Während einer Operation oder Behandlung kann die Halterung in besonders vorteilhafter Weise zur Zwischenlagerung des Werkzeugs verwendet werden, was zu besserer Ordnung und Übersichtlichkeit führt. Die Erfindung kann damit neben der Verpackung im eigentlichen Sinne durch die separate Haltevorrichtung eine eigenständige Halterung zur Verfügung stellen, die neben einer sicheren und stabilen Lagerung von Werkzeugen während des Transports in der jeweiligen Umverpackung außerdem eine Möglichkeit zur definierten Positionierung und sicheren Handhabung des Werkzeugs im Rahmen einer Aufbereitung (z.B. Sterilisierung) sowie bei einer Bereitstellung des Werkzeugs während einer Behandlung im OP ermöglicht. Dazu kann die Haltevorrichtung zusammen mit dem darin gehaltenen Werkzeug aus der Umverpackung genommen und aufgestellt werden.

Die Umverpackung kann insbesondere als Blister ausgebildet sein. Solche Blister sind günstig und können für nahezu beliebige Formen von Werkzeug und/oder Haltevorrichtung hergestellt werden. Vorzugsweise kann die Blisterverpackung eine Unterschale mit einer den Aufnahmeraum für das Werkzeug ausbildenden Vertiefung aufweisen. Eine solche Unterschale einfach mit einer zur lagestabilen Positionierung von Haltevorrichtung und Werkzeug ausreichenden Stabilität und Form ausgebildet sein. Durch eine an der Unterschale in bekannter Weise angeordnete Deckelfolie kann der Aufnahmeraum in der jeweils gewünschten Weise verschlossen sein, insbesondere hermetisch und/oder steril.

Nach einer weiteren Ausführungsform der Erfindung kann die Verpackung ein Trägerelement zum Beispiel nach Art eines Siebkorbs aufweisen. Dieses kann zusammen mit der Haltevorrichtung und dem daran gehaltenen Werkzeug, insbesondere mit einer Mehrzahl an Haltevorrichtungen mit dem jeweils daran gehaltenen Werkzeug, in der Verpackung angeordnet sein. Mittels eines solchen Trägerelements können mehrere Haltevorrichtung mit darin gehaltenen Werkzeugen besonders einfach und gleichzeitig gehandhabt werden, zum Beispiel einer Sterilisierung zugeführt und/oder auf einem Instrumentierungstisch abgestellt werden.

Nach einer weiteren Ausführungsform der Erfindung kann die Haltevorrichtung kodiert sein, beispielsweise indem sie farbig ausgebildet ist. Auf diese Weise kann mit der Erfindung besonders einfach ein System bereitgestellt werden, mit dem nicht nur unterschiedliche Werkzeuge für einen Nutzer einfach unterscheidbar kodiert sein können, sondern darüber hinaus eine solche Kodierung oder Kennzeichnung bestimmter Werkzeuge auch nach den Entpacken des Werkzeugs aus der Verpackung dem jeweiligen Werkzeug zugeordnet beibehalten werden kann. Insbesondere sind so bereits entpackte und für eine Verwendung auf einem Instrumentierungstisch oder in einer Sterilisationseinrichtung befindliche Werkzeuge für den Anwender besonders einfach und sicher zu erkennen. Eine Identifikation unterschiedlicher Werkzeug eund/oder Haltevorrichtung kann vorzugsweise über unterschiedliche Farbgebungen der Haltevorrichtungen erfolgen, beispielsweise im Hinblick auf deren Indikation oder auf bestimmte Produktgruppen, etc. Auf diese Weise können Fehlkombinationen von Werkzeugen durch die Farbgebung der insbesondere als Kunststoffspritzteil ausgebildeten Haltevorrichtung in und außerhalb der Verpackung vermieden werden.

Nach einer weiteren Ausführungsform der Erfindung kann die Haltevorrichtung als Kunststoffformteil ausgebildet sein. Wenn sie nach einer Fortbildung der Erfindung als Mehrkomponentenspritzguss ausgebildet ist, kann es zum Beispiel mit einer besonders rutschfesten Standfläche versehen sein. Dies ermöglicht eine stabile Anordnung der Haltevorrichtung auf einem Instrumentiertisch und/oder einem Trägerelement wie einem Siebkorb während eines Aufbereitungsprozesses. Außerdem kann so die Handhabbarkeit der Haltevorrichtung bei der Entnahme des ungebrauchten Werkzeugs wie auch bei der Einführung eines gebrauchten Werkzeugs ungemein erleichtern werden.

Zusammenfassend kann man sagen, dass die Erfindung eine Werkzeug- oder Arbeitsendenhalterung zur Verfügung stellt, welche eine sichere und stabile Lagerung von Arbeitsenden während des Transports in der jeweiligen Umverpackung sicherstellt. Bei Bereitstellung, im Speziellen beim Kupplungsprozess, wird sichergestellt, dass die Arbeitsenden nur von resorbierbarem Material berührt werden. Die Erfindung bewirkt insbesondere die folgenden Vorteile:
- Die Haltevorrichtung / Verpackung bietet eine hohe Sicherheit für einen Anwender
- Die Haltevorrichtung / Verpackung ist sehr kompakt
- Die Haltevorrichtung / Verpackung bietet eine sehr gute Lagesicherung
- Eine Querkontamination mit sehr hoher Sicherheit verhindert werden

Weitere Merkmale und Vorteile der vorliegenden Erfindung ergeben sich aus der folgenden beispielhaften und nicht beschränkenden Beschreibung der Erfindung anhand von Figuren. Diese sind lediglich schematischer Natur und dienen nur dem Verständnis der Erfindung. Dabei zeigen:
Fig. 1 eine perspektivische Ansicht einer Ausführungsform einer Haltevorrichtung nach der Erfindung mit einem darin gehaltenen Werkzeug,
Fig. 2 eine perspektivische Ansicht der Ausführungsform der Figur 1 vorbereitet zum Koppeln des Werkzeugs,
Fig. 3 eine perspektivische Ansicht der Haltevorrichtung der Figuren 1 und 2 beim Koppeln des darin gehaltenen Werkzeugs,
Fig. 4 eine weitere perspektivische Ansicht der Haltevorrichtung der Figuren 1 und 2 beim Koppeln des darin gehaltenen Werkzeugs,
Fig. 5 eine perspektivische Darstellung des mit einem Griffstück gekoppelten Werkzeugs,
Fig. 6 eine perspektivische Darstellung einer Verpackung nach der Erfindung,
Fig. 7 eine vergrößerte Detailansicht der Haltevorrichtung und
Fig. 8 eine Schnittansicht der Haltevorrichtung in Richtung quer zur Längsachse.

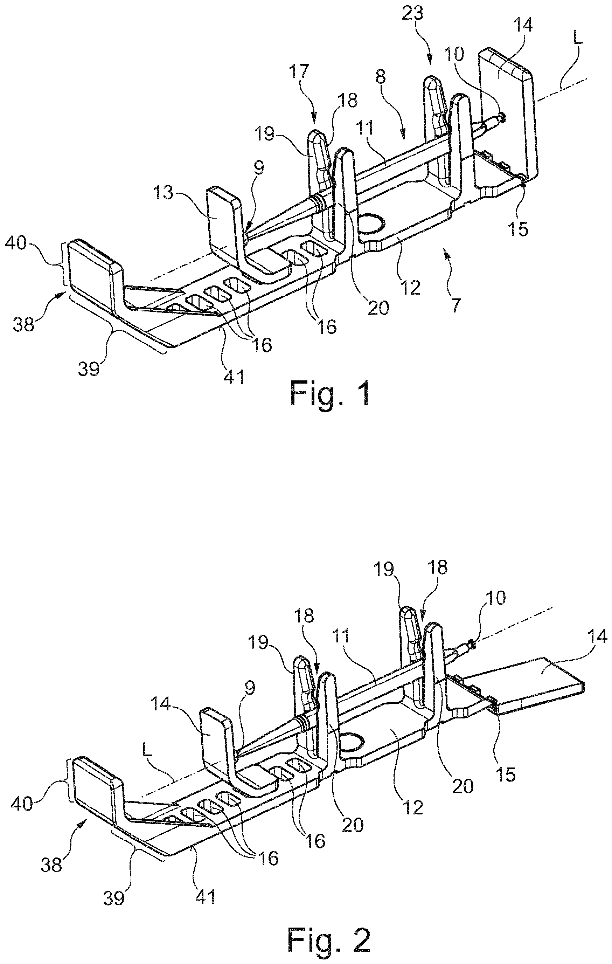

Figur 1 zeigt eine Haltevorrichtung 7 nach der Erfindung mit einem darin angeordnetem Werkzeug 8. Das Werkzeug 8 ist im vorliegenden Ausführungsbeispiel ein Bohrer 8, an dessen distalen Ende ein Arbeitsende 9 in Form eines Bohrkopfes 9 und an dessen proximalen Ende eine Koppelstruktur 10 zum Anordnen des Werkzeugs 8 in einer Werkzeugaufnahme 26 einer Antriebsgriffeinheit 27 ausgebildet sind. Zwischen dem Arbeitsende 9 und der Koppelstruktur 10 weist das Werkzeug 8 einen langgestreckten Werkzeugschaft 11 auf.

Die Haltevorrichtung ist als Kunststoffformteil ausgebildet und weist eine Grundplatte 12 auf. Mittig der Grundplatte 12 ist eine erste Haltestruktur 17 ausgebildet. Diese umfasst zwei einander gegenüberliegende und zwischen sich einen Aufnahmeschlitz 18 für den Werkzeugschaft 11 ausbildende und zueinander parallel Haltearme 19, 20, die beide zur Grundplatte 12 im Wesentlichen orthogonal angeordnet sind. Benachbart zur ersten Haltestruktur 17 ist eine zweite Haltestruktur 23 ausgebildet, die im Wesentlichen der ersten Haltestruktur 17 gleicht und daher nicht weiter beschrieben wird.

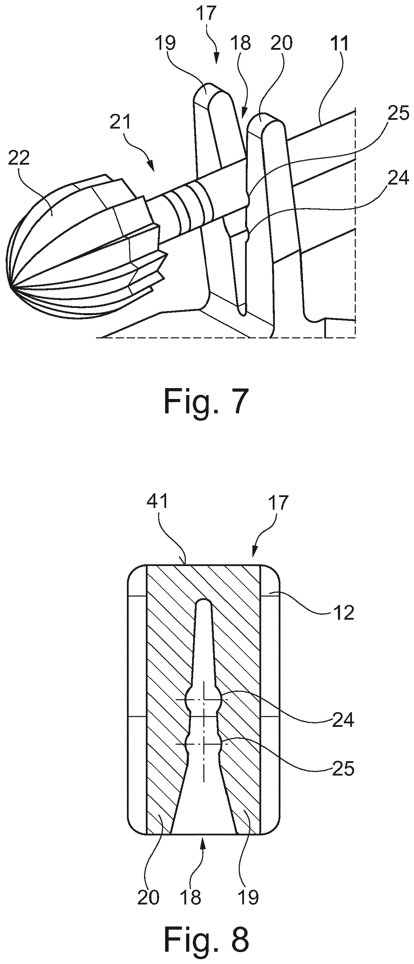

An dem distalen (arbeitsendenseitigen) Ende der Grundplatte 12 ist an dieser eine Schutzlasche 38 angeordnet. Die Schutzlasche 38 ragt von der Grundplatte 12 zunächst mit einem Schrägabschnitt 39 in einem schrägen Winkel (hier von etwa 45°) ab und ist dann um einen weiteren Winkel zu einem Endabschnitt 40 gebogen, so dass ihr von der Grundplatte 12 abgewandtes Ende in etwa quer zu dieser angeordnet ist. Wie insbesondere Figur 8 zeigt, ragt die Schutzlasche 38 derart weit von der Grundplatte 12 ab, dass das jeweilige distale Ende des Arbeitsendes 9 berührungsfrei abgedeckt ist. Diese Ausgestaltung der Schutzlasche 38 schafft eine gute Griffmöglichkeit in Form des Schrägabschnitts 39 zum Ergreifen durch einen Nutzer, wobei das Arbeitsende 9 verdeckt ist, so dass dessen Kontaktierung durch den Nutzer sicher verhindert und damit ein Verletzungsrisiko minimiert werden kann.

In der Grundplatte 12 sind zwischen der ersten Haltestruktur 17 und der distalen Schutzlasche 38 mehrere durchgehende Ausnehmungen 16 ausgebildet. Diese dienen einer Aufnahme und Positionierung eines Anschlags 13, der mit einem in den Figuren nicht dargestellten Zapfen form- und/oder kraftschlüssig in eine der Ausnehmungen 16 eingreift und derart an der Grundplatte 12 fixiert ist. Der Anschlag 13 besteht vollständig aus einem resorbierbaren Material und bildet eine Anlage für das Arbeitsende 9 des Werkzeugs 8 aus, mittels der dieses in Richtung der Längsachse L seines Werkzeugschafts 11 lagepositioniert ist. Am proximalen (koppelstrukturseitigen) Ende der Grundplatte 12 ist ein weiterer Anschlag 14 mittels einer Sollbruchstelle 15 stoffschlüssig daran angeordnet. Der weitere Anschlag 14 bildet eine Abdeckung für die Koppelstruktur 10 des Werkzeugs 8 aus.

Figur 7 zeigt die Haltestruktur 17 der Haltevorrichtung 7 in einer vergrößerten Detailansicht mit einem anderen Werkzeug 21, hier in Form eines Fräsaufsatzes 21. Der Fräsaufsatz 21 weist wie der Bohrer 8 der Figuren 1 bis 6 eine Koppelstruktur 10 und einen Werkzeugschaft 11 auf und unterscheidet sich vom Bohrer 8 durch sein als Fräskopf 22 ausgebildetes distales Arbeitsende 22. Dennoch können durch die Erfindung der Bohrer 8 und der Fräskopf 21 in gleichen Haltevorrichtungen 7 aufgenommen werden. Die einander zugewandten Seitenflächen der Haltearme 19, 20 sind mit jeweils zwei übereinander angeordneten Einbuchtungen 24, 25 von unterschiedlicher Größe versehen, jeweils zur Aufnahme von Werkzeugschäften 11 unterschiedlichen Durchmessers (siehe auch Figur 8, die die zweite Haltestruktur 17 in einer Schnittansicht quer zur Längsachse des Werkzeugs 8, 21 zeigt). Die Haltearme 19, 20 weisen gewisse elastische Federeigenschaften auf und können relativ zueinander eine Federbewegung ausführen, so dass sich der Schlitz 18 bei einem Einbringen eines Werkzeugs 8, 21 aufweiten kann und die Haltearme 19, 20 in ihre ursprüngliche Position zurückfedern, sobald der Schaft 11 des entsprechenden Werkzeugs 8, 21 in der Einbuchtung 24, 25 angeordnet ist. Auf diese Weise ist das Werkzeug 8, 21 sicher in der Haltevorrichtung 7 gehalten. Es kann insbesondere zwischen den Haltearmen 19, 20 geklemmt gehalten sein.

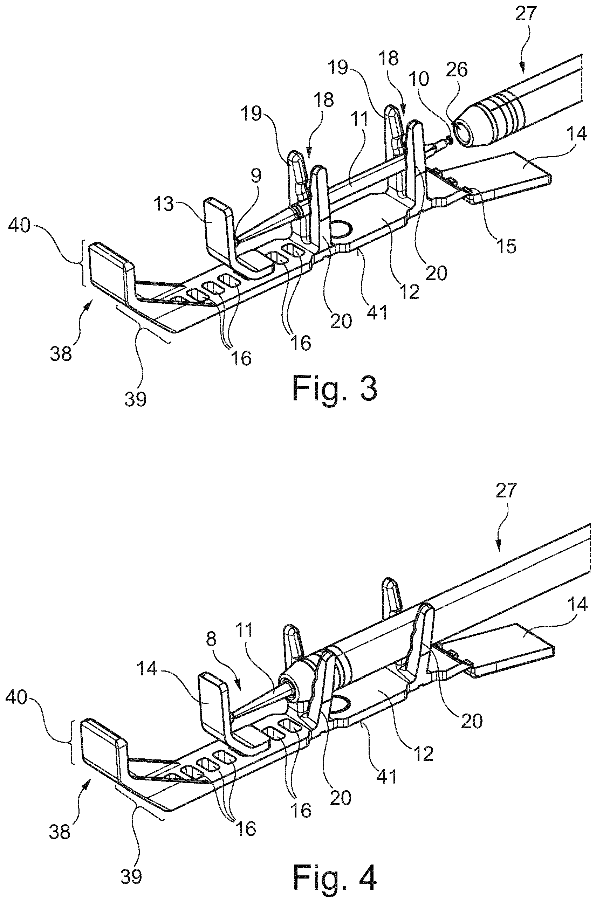

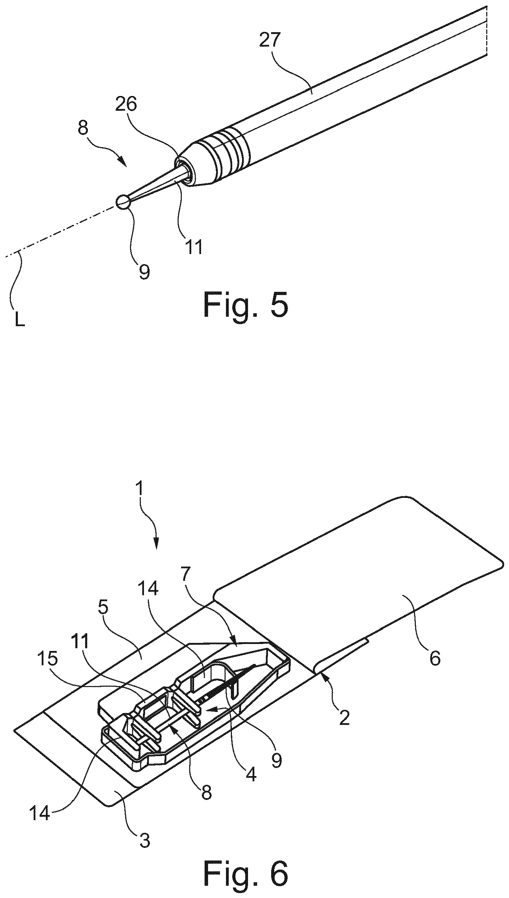

Figur 6 zeigt eine Ausführungsform einer Verpackung 1 nach der Erfindung. Diese umfasst eine Umverpackung 2 in Form eines Blisters 2 mit einer Unterschale 3 mit einer darin ausgebildeten Vertiefung 4 und einem die Vertiefung 4 umgebenden Rand 5. Die Umverpackung 2 umfasst außerdem eine an dem umlaufenden Rand 5 der Unterschale 3 angeordnete und die Vertiefung 4 hermetisch dicht verschließende Deckelfolie 6, die in den Figuren 1 und 3 jeweils in einem teilweise geöffneten Zustand gezeigt ist. In der Vertiefung 4 der Umverpackung 2 der Figur 1 ist eine Haltevorrichtung 7 nach der Erfindung mit einem darin gehaltenen Werkzeug 8 als Beispiel für ein verpacktes Produkt angeordnet.

Die Haltevorrichtung 7 ist zur Aufnahme und zum Halten von unterschiedlichen Werkzeugen 8, 21 geeignet ist, solange diese eine im Wesentlichen ähnliche Grundform mit einem Schaft 11 und einer Koppelstruktur 10 am proximalen Ende aufweisen und sich eigentlich nur hinsichtlich ihres jeweiligen Arbeitsendes 9, 22 unterscheiden. Des Weiteren ist die Haltevorrichtung 7 neben der Lagefixierung des Werkzeugs 8, 21 in einem verpackten Zustand, also in dem in Figur 6 gezeigten Zustand, als separat von der Umverpackung 2 nutzbare Haltevorrichtung genutzt zu werden, um das Werkzeug abzustellen, beispielsweise bei einer Bereitstellung im Rahmen einer OP oder bei einer Sterilisierung. Die Unterseite 41 der Grundplatte 12, 30 bildet dazu eine Standfläche 41, auf der die Haltevorrichtung 7 mit einem darin gehaltenen Werkzeug stabil abstellbar ist.

Anhand der Figuren 1 bis 5 kann die Verwendung der Haltevorrichtung 7 im Rahmen des Einbringens des Werkzeugs 8 in die Werkzeugaufnahme 26 einer Griffeinheit 27 nachvollzogen werden. Figur 1 zeigt die Haltevorrichtung 7 mit dem darin gehaltenen Werkzeug 8 im ursprünglichen verpackten Zustand. Dabei liegt das Arbeitsende 9 an dem Anschlag 13 an, so dass das Werkzeug 8 in Richtung der Schaftlängsachse L lagefixiert ist und insbesondere nicht in die distale Richtung, also in Richtung des Anschlags 13 verschoben werden kann. Figur 2 zeigt die Haltevorrichtung im weiteren Verlauf der Werkzeugentnahme. Der weitere proximale Anschlag 14 wurde unter Versagen der Sollbruchstelle 15 abgeknickt, so dass die proximale Koppelstruktur 10 des Werkzeugs freigegeben und zum Koppeln mit der Werkzeugaufnahme 26 zugänglich ist. Figur 3 zeigt, wie das Griffstück 27 aus der proximalen Richtung an die Koppelstruktur 10 herangeführt wird. Figur 4 zeigt schließlich den weiteren Verlauf des Koppelns, wobei das Griffstück 27 weiter in Richtung distal in die Haltevorrichtung 7 eingerückt wurde. Dabei federn die Haltearme 19, 20 beider Haltestrukturen 17, 23 nach außen aus. Gleichzeitig wird das Werkzeug 8 mit seinem Arbeitsende 9 gegen den aus einem resorbierbaren Material bestehenden Anschlag 13 gedrückt und kann sich nicht in distaler Richtung verlagern. Nach vollständigem Einrasten der Koppelstruktur 10 in die Werkzeugaufnahme 26 wird das Werkzeug 8 aus der Haltevorrichtung 7 entnommen.

### Bezugszeichenliste

- 1: Verpackung
- 2: Verpackung / Umverpackung
- 3: Unterschale
- 4: Vertiefung, Aufnahmeraum
- 5: Rand
- 6: Foliendeckel
- 7: Haltevorrichtung
- 8: Werkzeug, Bohraufsatz
- 9: Arbeitsende, Bohrkopf
- 10: Koppelstruktur
- 11: Werkzeugschaft
- 12: Grundplatte
- 13: Anschlag, distaler Anschlag
- 14: weiterer Anschlag, proximaler Anschlag
- 15: Sollbruchstelle
- 16: Ausnehmung
- 17: Haltestruktur
- 18: Aufnahmeschlitz
- 19: Haltearm, Klemmarm
- 20: Haltearm, Klemmarm
- 21: Werkzeug, Fräsaufsatz
- 22: Arbeitsende, Fräskopf
- 23: Haltestruktur
- 24: Einbuchtung
- 25: Einbuchtung
- 26: Werkzeugaufnahme
- 27: Antriebsgriffeinheit
- 38: Schutzlasche
- 39: Schrägabschnitt
- 40: Endabschnitt
- 41: Standfläche

## Patentansprüche

1. Verpackung (1), insbesondere Sterilverpackung (1), mit:
einer Haltevorrichtung (7) zum lagebestimmten Halten eines medizintechnischen Werkzeugs (8, 21) mit einem Werkzeugschaft (11) mit einem vor anwenderseitigen Kontakt zu schützenden Arbeitsende (9, 22) in einem Aufnahmeraum (4) der Verpackung, und
einer den Aufnahmeraum (4) für die Haltevorrichtung (7) und das darin gehaltene Werkzeug (8, 21) ausbildenden Umverpackung,
wobei die Haltevorrichtung (7) Haltemittel (17, 23) zur positionsbestimmten Aufnahme des Werkzeugs (8, 21) und einen Anschlag (13) für das Arbeitsende (9, 22) zum Positionieren des Werkzeugs (8, 21) in Richtung des Werkzeugschafts (11) aufweist,
**dadurch gekennzeichnet, dass**
der Anschlag (13) zumindest teilweise oder abschnittsweise aus einem resorbierbaren Material besteht.

2. Verpackung (1) nach Anspruch 1, **dadurch gekennzeichnet, dass** der Anschlag (13) vollständig aus einem resorbierbaren Material besteht.

3. Verpackung (1) nach Anspruch 1 oder 2, **dadurch gekennzeichnet, dass** der Anschlag (13) separat von der Haltevorrichtung (7) ausgebildet ist.

4. Verpackung (1) nach einem der vorstehenden Ansprüche, **dadurch gekennzeichnet, dass** der Anschlag (13) in einer Mehrzahl unterschiedlicher Positionen an der Haltevorrichtung (7), insbesondere in unterschiedlichen Positionen in Richtung des Werkzeugschafts (11), anordbar ist.

5. Verpackung (1) nach einem der vorstehenden Ansprüche, **dadurch gekennzeichnet, dass** diese einen weiteren Anschlag (14) für eine dem Arbeitsende (9, 22) gegenüberliegende Koppelstruktur (29) des Werkzeugs (9, 22) zum Positionieren des Werkzeugs (8, 21) in Richtung des Werkzeugschafts (11) aufweist.

6. Verpackung (1) nach Anspruch 5, **dadurch gekennzeichnet, dass** der weitere Anschlag (14) mittels einer Sollbruchstelle (15) mit der Haltevorrichtung (7) verbunden ist und durch Brechen der Sollbruchstelle (15) von der Haltevorrichtung (7) entfernbar ist.

7. Verpackung (1) nach einem der vorstehenden Ansprüche, **dadurch gekennzeichnet, dass** die Haltevorrichtung (7) eine Grundplatte (12) aufweist, die eine Standfläche (41) zum lagestabilen Aufstellen der Haltevorrichtung (7) mit einem darin gehaltenen Werkzeug (8, 21) ausbildet.

8. Verpackung (1) nach Anspruch 7, **dadurch gekennzeichnet, dass** die Haltemittel zumindest zwei voneinander beabstandete Haltestrukturen (17, 23) zum lagebestimmten Positionieren des Werkzeugs (8, 21) in einer Richtung quer zum Werkzeugschaft (11) aufweist.

9. Verpackung (1) nach Anspruch 8, **dadurch gekennzeichnet, dass** zumindest eine der Haltestrukturen (17, 23), insbesondere beide Haltestrukturen (17, 23), jeweils zwei einander gegenüberliegende Haltearme (19, 20) aufweist bzw. aufweisen, zwischen denen ein Schlitz (18) zur gegebenenfalls klemmenden Aufnahme und Halterung des Werkzeugs (8, 21) ausgebildet ist, wobei insbesondere sich der Schlitz (18) von proximal der Grundplatte (12) in Richtung distal der Grundplatte (12) aufweitet.

10. Verpackung (1) nach einem der Ansprüche 7 bis 9, **dadurch gekennzeichnet, dass** in der Grundplatte (12) ein Mehrzahl von zueinander beabstandeten Ausnehmungen (16) ausgebildet ist und der Anschlag (13) für das Arbeitsende (9, 22) einen dazu passenden Zapfen aufweist, so dass der Anschlag (13) durch Einbringen des Zapfens in eine der Ausnehmungen (16) in unterschiedlichen Positionen an der Grundplatte (12) anordbar ist.

11. Verpackung (1) nach einem der vorstehenden Ansprüche, **dadurch gekennzeichnet, dass** die Umverpackung (2) als Blister ausgebildet ist, mit einer Unterschale (3), die eine den Aufnahmeraum (4) ausbildende Vertiefung (4) aufweist, und mit einer daran angeordneten und den Aufnahmeraum verschließenden Deckelfolie (6).

## Claims

1. Packaging (1), in particular sterile packaging (1), comprising:
a holding device (7) for location-specific holding of a medical tool (8, 21) comprising a tool shaft (11) with a working end (9, 22), which is to be protected against user-side contact, in a receiving space (4) of the packaging, and
an outer packaging forming a receiving space (4) for the holding device (7) and the tool (8, 21) held therein,
wherein the holding device (7) has holding means (17, 23) for position-specific reception of the tool (8, 21), and a stop (13) for the working end (9, 22) for positioning the tool (8, 21) in the direction of the tool shaft (11),
**characterised in that**
the stop (13) consists at least partially or in sections of a resorbable material.

2. Packaging (1) according to claim 1, **characterised in that** the stop (13) consists entirely of a resorbable material.

3. Packaging (1) according to claim 1 or 2, **characterised in that** the stop (13) is formed separately from the holding device (7).

4. Packaging (1) according to any of the preceding claims, **characterised in that** the stop (13) can be arranged in a plurality of different positions on the holding devices (7), in particular in different positions in the direction of the tool shaft (11).

5. Packaging (1) according to one of the preceding claims, **characterised in that** it has a further stop (14) for a coupling structure (29) of the tool (9. 22) opposite the working end (9, 22) for positioning the tool (8, 21) in the direction of the tool shaft (11).

6. Packaging (1) according to claim 5, **characterised in that** the further stop (14) is connected to the holding device (7) by means of a predetermined breaking point (15) and can be removed from the holding device (7) by breaking the predetermined breaking point (15).

7. Packaging (1) according to any of the preceding claims, **characterised in that** the holding device (7) has a base plate (12) which forms a standing surface (41) for setting up the holding device (7), with a tool (8, 21) held therein, in a stable position.

8. Packaging (1) according to claim 7, **characterised in that** the holding means has at least two spaced-apart holding structures (17, 23), for positioning of the tool (8, 21) in a specific location in a direction transverse to the tool shaft (11).

9. Packaging (1) according to claim 8, **characterised in that** at least one of the holding structures (17, 23), in particular both holding structures (17, 23), each has or have two holding arms (19, 20) opposite each other, between which a slot (18) is formed for optional clamped reception and holding of the tool (8, 21), wherein in particular the slot (18) widens from the proximal of the base plate (12) in the distal direction of the base plate (12).

10. Packaging (1) according to one of claims 7 to 9, **characterised in that** a plurality of spaced-apart recesses (16) is formed in the base plate (12) and the stop (13) for the working end (9, 22) has a matching pin, so that the stop (13) can be arranged in different positions on the base plate (12) by inserting the pin in one of the recesses (16).

11. Packaging (1) according to any of the preceding claims, **characterised in that** the outer packaging (2) is formed as a blister, with a lower shell (3), which has an indentation (4) forming the receiving space (4), and with a cover film (6) arranged thereon and sealing the receiving space.

## Revendications

1. Emballage (1), en particulier un emballage stérile (1), comprenant :
un dispositif de retenue (7) destiné à maintenir dans une position déterminée un instrument utilisé en technique médicale (8, 21) muni d'une tige d'instrument (11) comportant une extrémité de travail (9, 22) à protéger des contacts côté utilisateur dans un espace de réception (4) de l'emballage, et
un suremballage formant l'espace de réception (4) pour le dispositif de retenue (7) et l'instrument qui y est contenu,
le dispositif de retenue (7) présente un moyen de retenue (17, 23) destiné à recevoir dans une position déterminée l'instrument (8, 21) et une butée (13) pour l'extrémité de travail (9, 22) destinée à positionner l'instrument (8, 21) en direction de la tige d'instrument (11),
**caractérisé en ce que**
la butée (13) se compose au moins partiellement ou par endroits d'un matériau résorbable.

2. Emballage (1) selon la revendication 1, **caractérisé en ce que** la butée (13) se compose entièrement d'un matériau résorbable.

3. Emballage (1) selon la revendication 1 ou 2, **caractérisé en ce que** la butée (13) est formée séparément du dispositif de retenue (7).

4. Emballage (1) selon l'une quelconque des revendications précédentes, **caractérisé en ce que** la butée (13) peut être disposée selon une pluralité de positions différentes sur le dispositif de retenue (7), en particulier dans différentes positions en direction de la tige d'instrument (11).

5. Emballage (1) selon l'une quelconque des revendications précédentes, **caractérisé en ce que** celui-ci présente une autre butée (14) pour une structure de couplage (29) opposée à l'extrémité de travail (9, 22) de l'instrument (9, 22) pour positionner l'instrument (8, 21) en direction de la tige d'instrument (11).

6. Emballage (1) selon la revendication 5, **caractérisé en ce que** l'autre butée (14) est reliée au moyen d'un point de rupture (15) au dispositif de retenue (7) et en cassant le point de rupture (15) est amovible du dispositif de retenue (7).

7. Emballage (1) selon l'une quelconque des revendications précédentes, **caractérisé en ce que** le dispositif de retenue (7) présente une plaque de base (12), qui forme une surface d'appui (41) pour installer en position stable le dispositif de retenue (7) avec un instrument (8, 21) contenu dans celui-ci.

8. Emballage (1) selon la revendication 7, **caractérisé en ce que** le moyen de retenue présente au moins deux structures de retenue (17, 23) espacées les unes des autres pour positionner dans une position déterminée l'instrument (8, 21) en direction transversale par rapport à la tige d'instrument (11).

9. Emballage (1) selon la revendication 8, **caractérisé en ce qu'**au moins l'une des structures de retenue (17, 23), en particulier les deux structures de retenue (17, 23), présente ou présentent respectivement deux bras de retenue (19, 20) opposés l'un à l'autre, entre lesquels une fente (18) est formée pour le cas échéant la réception par serrage et la fixation de l'instrument (8, 21), en particulier la fente (18) s'élargissant du côté proximal de la plaque de base (12) en direction distale de la plaque de base (12).

10. Emballage (1) selon l'une des revendications 7 à 9, **caractérisé en ce que** dans la plaque de base (12) est formée une pluralité d'évidements (16) espacés les uns des autres et la butée (13) présente pour l'extrémité de travail (9, 22) un tourillon adapté à celle-ci, de sorte que la butée (13) peut être disposée par introduction du tourillon dans l'un des évidements (16) dans différentes positions sur la plaque de base (12).

11. Emballage (1) selon l'une quelconque des revendications précédentes, **caractérisé en ce que** le suremballage (2) est formé en tant que plaquette thermoformée, d'une partie inférieure (3), qui présente un renfoncement (4) formant l'espace de réception (4), et d'une feuille de recouvrement (6) disposée sur celui-ci et scellant l'espace de réception.
